# EUROPEAN PATENT SPECIFICATION

(11) **EP 0 762 906 B1**
(45) Date of publication and mention of the grant of the patent: **28.08.2002**
(21) Application number: 95919899.5
(22) Date of filing: 22.05.1995
(51) Int. Cl.: A61M 25/01, A61M 16/04

(54) **APPARATUS FOR PRODUCING A TRANSLARYNGEAL TRACHEOSTOMY**
EIN INSTRUMENT ZUR DURCHFÜHRUNG EINER TRANSLARYNGALEN TRACHEOTOMIE
APPAREIL POUR PRATIQUER UNE TRACHEOTOMIE TRANSLARYNGIENNE

(30) Priority: 23.05.1994 IT MI941043
(43) Date of publication of application: 19.03.1997
(73) Proprietor: Mallinckrodt Inc., St. Louis, Missouri 63134 (US)
(72) Inventor: FANTONI, Antonio, I-20145 Milano (IT)
(74) Representative: Modiano, Guido, Dr.-Ing.
(86) International application number: US9506403
(87) International publication number: WO95032017

(56) References cited:
- US-A- 4 444 185
- US-A- 4 471 778
- US-A- 4 677 978
- US-A- 4 795 430
- US-A- 4 889 112
- US-A- 5 055 107
- US-A- 5 186 168
- US-A- 5 217 005
- US-A- 5 247 942

## Description

The present invention relates to an apparatus for producing a translaryngeal tracheostomy.

It is known that tracheotomy is normally performed in an operating theater in aseptic conditions with the aid of an assistant, an anesthesiologist, and a scrub nurse.

The patient is placed in the dorsal position, with the head in hyperextension with a cushion under the shoulders to exteriorize the laryngotracheal region.

Various percutaneous methods and apparatus are known for intubating an ostomy formed by endoscopic techniques. One method and apparatus is disclosed by U.S. patent 4,495,430 to Quinn et al. The apparatus employed therein provided a multilumen feeding tube, one tube for dispensing liquids and one for inflating an inflatable cuff surrounding the tube. The multilumen tube is equipped with a suture loop that is employed to pull the tube into the esophagus in retrograde manner. However, first a gastrostomy is performed to provide an opening into which a suture drawn up, through and out of the mouth of the patient. The suture is then tied to the loop of the feeding tube and is then withdrawn thereby pulling the tube into position in the stomach and through the opening to provide a feeding tube that is sealed in position by means of inflating the cuff.

In another example of a percutaneous or non-dissection opening into a body cavity there described in US Patent 4,471,778 a needle that is structurally weakened along a longitudinal line and includes elements operable to split the needle along the line to provide a longitudinal line. An elongated dilator slidably fits through a side opening in a flexible tube and extended through the distal end of the tube. A leader attached to the distal end of the dilator is adapted for injection through the bore of the needle, the slot in the needle enabling lateral separation of the leader for removal of the needle from the body cavity. The dilator is adapted to be forcibly inserted into the body cavity along the path defined by the leader and is thereafter removable through the side opening in the tube. The tube is thereafter inserted into the body cavity.

To perform tracheotomy, a cutaneous incision is performed that includes the skin, the subcutaneous cell tissue, and the cutaneous muscle. The incision is approximately 4 cm long and is performed along a horizontal line at the upper margin of the jugular fossa; optionally, the incision can also be performed vertically along the median line, starting from the cricoid cartilage and continuing downward for 3-4 cm.

The vertical incision has the advantage that it does not cross the surface veins, but it leads to a more conspicuous scar.

In conventional techniques, therefore, in order to perform tracheotomy it is necessary to perform an actual operation, with all the difficulties and contraindications typical of this procedure.

The aim of the present invention is to provide an apparatus for producing a translaryngeal tracheostomy that allows, instead of performing an operation, to insert, according to a new technique, a tracheostomy tube which in practice is bloodless and most of all does not require the incision of skin or muscle tissues, thus eliminating the times and problems linked to cicatrization.

Within the scope of the above aim, a particular object of the invention is to provide an apparatus that allows to apply the tracheostomy tube from the inside outward, radically modifying the techniques used so far.

Another object of the present invention is to provide an apparatus that allows to produce a tracheostomy, avoiding an operation and all complications such as hemorrhages, infections, large and unaesthetic scars, and the like, which are normally linked to an operation.

Another object of the present invention is to provide an apparatus that allows to apply the tracheostomy tube in considerably less time than conventional methods.

In accordance with the invention, there is provided an apparatus for producing a translaryngeal tracheostomy as defined in appended claim 1. Further embodiments of the invention are defined in the dependent claims.

The characteristics and advantages will become apparent from the description of some preferred but not exclusive embodiments of an apparatus for producing a translaryngeal tracheostomy, illustrated only by way of nonlimitative example in the accompanying drawings, wherein:
figure 1 is a schematic sectional exploded view of the tracheostomy tube and of the parts related to it;
figure 2 is a view of the tracheostomy tube, assembled for insertion, in a first embodiment;
figure 3 is a view of the tracheostomy tube with a rigid penetration cone;
figure 4 is a view of the tracheostomy tube in which the penetration element is formed by multiple cones;
figure 5 is a view of the initial step for the insertion of a rigid cannula with a bronchoscope;
figure 6 is a view of the step for the insertion of the trocar;
figure 7 is a view of the step for the insertion of the traction wire;
figure 8 is a view of the insertion of the traction wire and of the exit of its end from the oral cavity;
figure 9 is a view of the step for connecting the traction wire to the tracheostomy tube;
figure 10 is a view of the exit of the tracheostomy tube from the interannular region of the trachea;
figure 11 is a view of the removal of the penetration element of the tracheostomy tube;
figure 12 is a view of the arrangement of the tracheostomy tube in the trachea;
figure 13 is a schematic perspective view of a tool for pulling the traction wire;
figure 14 is a view of the tool assembled at the end of the step for the extraction of the penetration element of the tracheostomy tube.

With reference to the above figures, the apparatus for producing a translaryngeal tracheostomy comprises a rigid cannula 1, provided with an inflatable sleeve 2, that can be inserted in the trachea 3. The cannula 1 can be connected to the ventilation unit to keep the patient correctly ventilated.

The rigid cannula is provided, in a per se known manner, with an elastic plug 5 for inserting a bronchoscope 6 without allowing gas to escape, thus maintaining the continuity of the ventilation of the patient.

The bronchoscope 6 is placed inside the cannula 1 and allows to identify the interannular space, which is generally designated by the reference numeral 10. Lighting is performed by moving the cannula 1 forward towards the front wall of the trachea 3, so that the beam of light projected by the bronchoscope allows to identify the region 10 from outside by transillumination.

A trocar 11 can be inserted in the interannular region 10 and is inserted from outside towards the inside of the trachea, at a downward angle, towards the illuminated region, so that the needle enters, as shown in figure 6, the cannula 1, from which the bronchoscope 6 can now be removed.

The trocar 11 acts as guiding element for a traction wire 20 which is inserted so that by passing through the cannula 1 it can easily exit from the oral cavity with its end.

Once the traction wire has been positioned, the cannula 1 and the trocar 11 are removed, thus achieving the condition shown in figure 8.

The end of the traction wire 20 is inserted in a tracheostomy tube, generally designated by the reference numeral 30, which is constituted by a tubular element 31 which has an inflatable sleeve 32 at one end and, at the other end, a penetration element which is generally designated by the reference numeral 35.

The inflatable sleeve 32 is connected to an air supply tube 32a which during the insertion step, as clarified hereinafter, is placed inside the tubular element 31 and can subsequently be removed to connect it to the pumping element 32b.

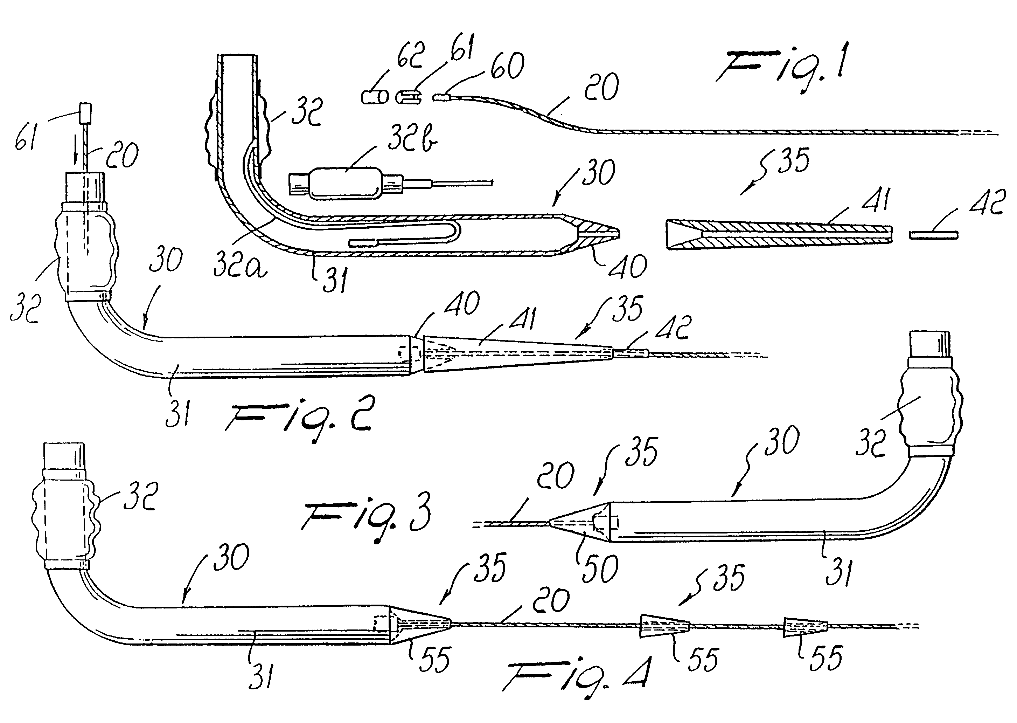

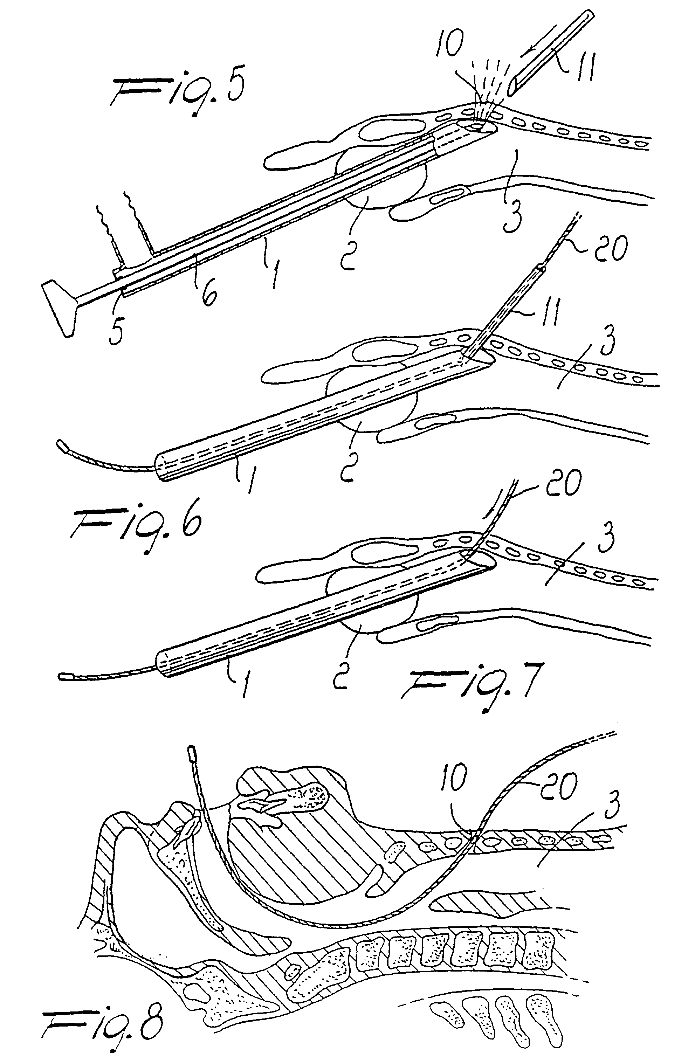

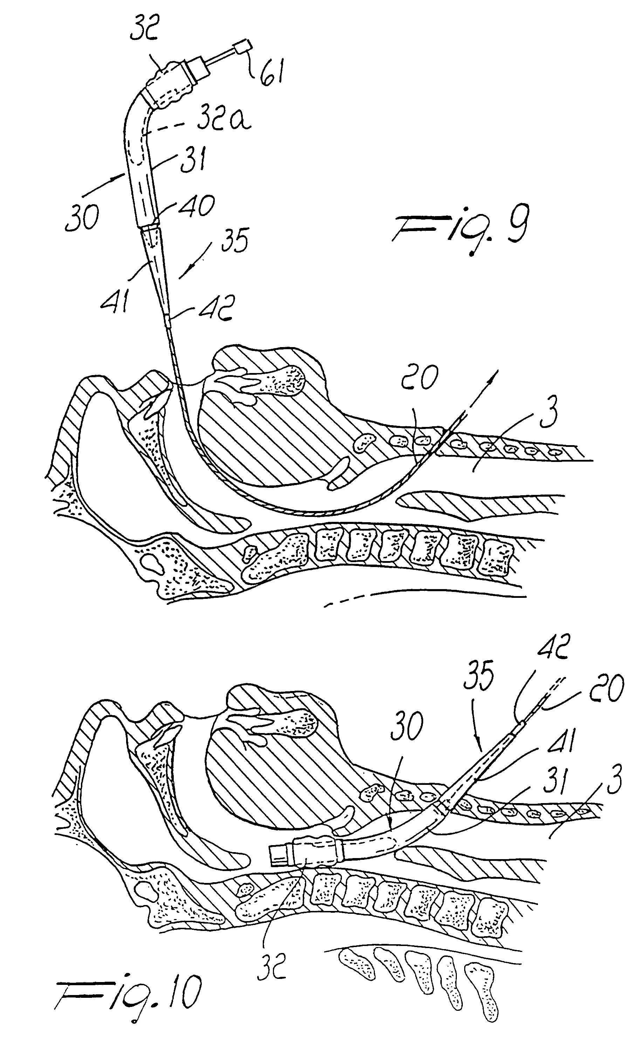

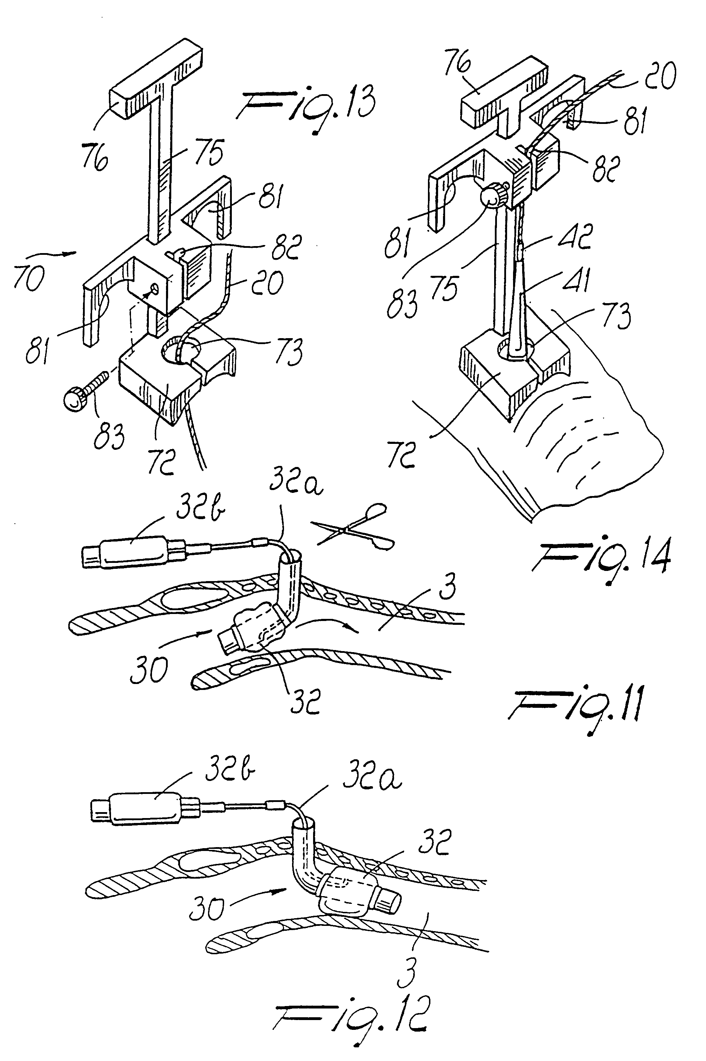

The penetration element 35 is constituted, as shown in figures 1 and 2, by a fixed cone 40 which is placed at the end of the tubular element 31 and on which a flexible cone 41 is associated, for example by gluing; said cone has a metal insert 42 at the penetration tip.

In this manner, one obtains a low-taper penetration element that produces a gradual divarication as it enters the interannular region.

The penetration element 35, as shown in figure 3, can also be obtained by means of a single rigid cone, optionally made of metal or covered with metal, designated by the reference numeral 50, which is located at the end of the tubular element 31 and has a larger taper angle, producing a faster type of divarication.

According to another embodiment of the penetration element 35, shown in figure 4, there are multiple cones 55 which have progressively larger dimensions and are arranged sequentially with respect to the traction wire 20 that joins them.

The traction wire 20 has, at the end where the tracheostomy tube 30 is located, enlargement means which have the purpose of preventing the disengagement of the traction wire 20 from the tracheostomy tube when traction is applied to the wire in order to make the penetration element 35 produce a gradual expansion of the interannular region 10, consequently causing the exit of the tracheostomy tube, which is inserted from the inside outward.

The enlargement means can be provided in various manners and can be constituted by a first retainer 60, which is fixed directly to the end of the wire and can be inserted in the trocar 11 during the initial step and on which a cup 61 and an elastic ring 62 are applied, preventing disengagement, once the wire has been inserted in the tracheostomy tube.

It is optionally also possible to provide an element that can be coupled to the first retainer 60 so as to increase its size and prevent extraction.

Furthermore, if one wishes to use a smaller trocar, it is possible to provide no enlarged portions on the wire and to couple to said wire, once it has been inserted in the tracheostomy tube, a small strip which can be folded in the shape of the letter U so as to in practice enlarge the end of the wire, preventing its accidental extraction, even when the traction wire is pulled.

Of course it is possible to use other elements that allow to enlarge the end of the traction wire after it has been inserted in the tracheostomy tube, such as for example a knot in the wire.

Once the penetration element has been made to exit from the interannular region 10 by means of the traction wire, the penetration element 35 is removed by cutting and the inflation duct 32a is connected to the pumping element 32b; after directing the loose end of the tracheostomy tube 30 towards the pulmonary region, the sleeve element 32 is inflated, thus allowing to ventilate the patient.

In this manner the tracheostomy tube is therefore applied from the inside outward and most of all without having to perform an operation that causes bleeding but by simply obtaining a gradual divarication of the interannular region where a very small hole has been formed by means of the trocar.

In order to facilitate the application of the tracheostomy tube, and in particular in order to be able to apply correct traction to the traction wire 20, a tool 70 is provided; said tool is shown in figures 13 and 14 and consists of a base body 71 which has a plate 72 that can rest at the outer surface of the trachea in the region where the wire 2 exits. The plate 72 is provided with a passage hole 73 which is larger than the diameter of the tracheostomy tube that is being used.

The plate 72 is connected to a guiding bar 75 which ends with an abutment cross-member 76 that can rest at the palm of the hand of the user.

A slider 80 is slideable on the guiding bar 75, is provided with grip hollows 81 for the fingers of the operator, and forms a seat 82 in which it is possible to insert the traction wire 20, which can be locked by virtue of locking means constituted for example by a screw 83.

In order to pull the traction wire 20, the operator must therefore fix the end of the traction wire that protrudes from the hole formed in the interannular region and then, by resting the palm of the hand on the crossmember 76, pull the traction wire by acting on the slider 80, so that he can easily extract the penetration element and therefore the tracheostomy tube as well.

In this manner, the plate 82 furthermore rests against the outer surface of the trachea, thus providing an abutment element that prevents lacerations in the region affected by the passage of the penetration element.

Finally, use of the above described tool allows the operator to apply traction gradually, accordingly avoiding forces that are excessive or otherwise dangerous to the patient.

From the above description it is thus evident that the invention achieves the intended aim and objects, and in particular the fact is stressed that an apparatus is provided which allows to produce a tracheostomy according to fully innovative methods, since the tracheostomy tube is inserted from the inside of the trachea outward, avoiding an operation which causes bleeding and in no way damaging the muscle and skin tissues.

The invention thus conceived is susceptible of numerous modifications and variations, all of which are within the scope of the inventive concept.

All the details may furthermore be replaced with other technically equivalent elements.

In practice, the materials employed, as well as the contingent shapes and dimensions, may be any according to the requirements.

## Claims

1. Apparatus for producing a translaryngeal tracheostomy, comprising: a trocar (11) that can be inserted in an interannular space of the trachea, from the outside inward; a traction wire (20) which can be inserted in said trocar to move the end of the wire so as to extend outside the oral cavity; a tracheostomy tube (30) which is provided with a substantially conical end (40) into which said end of said traction wire which is outside the oral cavity can be inserted; **characterized by** an enlargement means (61) for preventing disengagement of the traction wire (20) from said tracheostomy tube (30) which means (61) can be fixed to said end of said traction wire (20) aimed to be moved outside the oral cavity after said end has been inserted in said tracheoscomy tube (30) whereby said tracheoscomy (30) can be pulled into the trachea and to make it exit from said interannular space from the inside outward by means of traction applied to said traction wire (20) from the end opposite said tube (30).

2. Apparatus according to claim 1, **characterized in that** it comprises a rigid cannula (1) which is provided with an inflatable sleeve (2) for inserting a bronchoscope (6), said rigid cannula (1) being arrangeable so as to indicate said interannular space with its free end by transillumination with the beam of light projected by said bronchoscope (6).

3. Apparatus according to claim 1 **characterized in that** said tracheostomy tube (30) further comprises a tubular element (31), which has an inflatable sleeve (32) at one end and has a penetration element (35) at the other substantially conical end.

4. Apparatus according to claim 3 **characterized in that** said inflatable sleeve (32) is connected to an air supply tube (32a) which can be accommodated inside said tubular element (31) and can be extracted, when the penetration end of said tubular element (35) exits from the trachea, to connect it to a pumping element.

5. Apparatus according to claim 3, **characterized in that** said penetration element (35) further comprises a fixed cone (40) which is placed at the end of said tubular element, (35) a flexible cone (41) being associated on said fixed cone (40), and ending with a penetration tip that is constituted by a metal insert.

6. Apparatus according to claim 3, **characterized in that** said penetration element (35) is constituted by a single rigid cone (40) which is arranged at the end of said tubular element (31).

7. Apparatus according to claim 3, **characterized in that** said penetration element (35) is constituted by multiple cones (55) which have progressively larger dimensions and are arranged sequentially with respect to said traction wire (20) that links them.

8. Apparatus according to claim 1, **characterized in that** said enlargement means (61) are constituted by a first retainer (60) which is fixed directly to the end of said traction wire and can be inserted in said trocar, a cup-like element (61) being applicable to said first retainer.

9. Apparatus according to claim 8, **characterized in that** it further comprises an elastic ring (62) which can be interposed between said first retainer (60) and said cup like element (61).

10. Apparatus according to claim 1, **characterized in that** said enlargement means (61) is constituted by folding wire (20) into the shape of a letter U after its insertion into the tracheostomy tube (30) to prevent its extraction.

11. Apparatus according to claim 1, **characterized in that** said enlargement means (61) is constituted by a knot which is produced on said traction wire.

12. Apparatus according to claim 1, **characterized in that** it further comprises a tool (70) for pulling said traction wire (20), said tool (70) comprising a base body (71) which is provided with a plate (72) that can be rested at the outer surface of the trachea, said plate (72) forming a passage hole (73) for said penetration element (35), a guiding bar (75) extending from said plate (72) and ending with an abutment cross-member (76), a slider (80) being slidingly arranged on said guiding bar (75) and having means for fixing to said traction wire (20).

13. Apparatus according to claim 12, **characterized in that** said slider is further provided with grip hollows (81) for the fingers of the operator, said crossmember (76) being suitable to provide rest to the palm of the hand when applying traction to said traction wire (20).

## Patentansprüche

1. Gerät zum Durchführen einer translaryngealen Tracheostomie, mit einem Trokar (11), der von außen nach innen in einen Ringzwischenraum der Trachea einführbar ist; einem Zugdraht (20), der in den Trokar einführbar ist, um das Ende des Drahtes so zu bewegen, daß es aus der Mundhöhle hervorsteht; einer Trachealkanüle (30) mit einem im wesentlichen konischen Ende (40), in das das Ende des Zugdrahts, das außerhalb der Mundhöhle liegt, eingeführt werden kann, **gekennzeichnet durch** ein Verdickungsmittel (61) zum Verhindern, daß sich der Zugdraht (20) von der Trachealkanüle (30) löst, wobei das Mittel (61) an dem Ende des Zugdrahts (20) befestigt werden kann, das aus der Mundhöhle hinaus bewegt werden soll, nachdem das Ende in die Trachealkanüle (30) eingeführt worden ist, wodurch die Trachealkanüle in die Trachea gezogen werden kann, und um sie **durch** Zug, der von dem der Kanüle (30) abgewandten Ende aus auf den Zugdraht (20) ausgeübt wird, zu veranlassen, von innen nach außen aus dem Ringzwischenraum auszutreten.

2. Gerät nach Anspruch 1, **dadurch gekennzeichnet, daß** es eine starre Kanüle (1) mit einer aufblasbaren Muffe (2) zum Einführen eines Bronchoskops (6) hat, wobei die starre Kanüle (1) so angeordnet werden kann, daß sle durch Durchleuchtung mit dem von dem Bronchoskop (6) projizierten Lichtstrahl mit ihrem freien Ende den Ringzwischenraum anzeigt.

3. Gerät nach Anspruch 1, **dadurch gekennzeichnet, daß** die Trachealkanüle (30) ferner ein rohrförmiges Element (31) mit einer aufblasbaren Muffe (32) an einem Ende und einem Penetrationselement (35) an dem im wesentlichen konischen anderen Ende enthält.

4. Gerät nach Anspruch 3, **dadurch gekennzeichnet, daß** die aufblasbare Muffe (32) mit einem Luftzuführrohr (32a) verbunden ist, das in dem rohrförmtgen Element (31) aufgenommen und, wenn das Penetrationsende des rohrförmigen Elements (31) aus der Trachea austritt, herausgezogen werden kann, um es mit einem Pumpelement zu verbinden.

5. Gerät nach Anspruch 3, **dadurch gekennzeichnet, daß** das Penetrationselement (35) ferner einen festen Konus (40) enthält, der am Ende des rohrförmigen Elements (31) angeordnet ist, wobei ein flexibler Konus (41) auf dem festen Konus (40) befestigt ist und mit einer Penetrationsspitze endet, die aus einem Metalleinsatz besteht.

6. Gerät nach Anspruch 3, **dadurch gekennzeichnet, daß** das Penetrationselement (35) aus einem einzigen festen Konus (40) besteht, der am Ende des rohrförmigen Elements (31) angeordnet ist.

7. Gerät nach Anspruch 3, **dadurch gekennzeichnet, daß** das Penetrationselement (35) aus mehreren Konussen (55) besteht, die schrittweise größer werdende Abmessungen haben und bezüglich des Zugdrahts (20) nacheinander angeordnet sind, der sie verbindet.

8. Gerät nach Anspruch 1, **dadurch gekennzeichnet, daß** die Verdickungsmittel (61) aus einem ersten Halteelement (60) bestehen, das direkt an dem Ende des Zugdrahts befestigt und in den Trokar einführbar ist, wobei ein becherförmiges Element (61) auf das erste Halteelement aufsetzbar ist.

9. Gerät nach Anspruch 8, **dadurch gekennzeichnet, daß** es ferner einen elastischen Ring (62) enthält, der zwischen das erste Halteelement (60) und das becherförmige Element (61) eingesetzt werden kann.

10. Gerät nach Anspruch 1, **dadurch gekennzeichnet, daß** das Verdickungsmittel (61) gebildet wird, indem der Draht (20) in die Form eines U gebogen wird, nachdem er in die Trachealkanüle (30) eingeführt worden ist, um ein Herausziehen desselben zu verhindern.

11. Gerät nach Anspruch 1, **dadurch gekennzeichnet, daß** das Verdickungsmittel (61) aus einem Knoten besteht, der auf dem Zugdraht gebildet ist.

12. Gerät nach Anspruch 1, **dadurch gekennzeichnet, daß** es ferner ein Werkzeug (70) zum Ziehen des Zugdrahts (20) umfaßt, wobei das Werkzeug (70) enthält: einen Basiskörper (71) mit einer Platte (72), die an die Außenseite der Trachea angelegt werden kann, wobei die Platte (72) eine Druchgangsöffnung (73) für das Penetrationselement (35) ausbildet, eine Führungsschiene (75), die von der Platte (72) ausgeht und mit einem Anschlagquerelement (76) endet, ein Gleitelement (80), das verschiebbar auf der Führungsschiene (75) angeordnet ist und Mittel zum Befestigen an dem Zugdraht (20) hat.

13. Gerät nach Anspruch 12, **dadurch gekennzeichnet, daß** das Gleitelement ferner mit Griffvertiefungen (81) für die Finger der Bedienperson versehen ist, wobei das Querelement (76) geeignet ist, der Handfläche eine Auflage zu bieten, wenn auf den Zugdraht (20) Zug ausgeübt wird.

## Revendications

1. Dispositif pour réaliser une trachéotomie translaryngée, comprenant : un trocart (11) qui peut être inséré dans un espace inter-annulaire de la trachée artère, de l'extérieur vers l'intérieur ; un fil de traction (20) qui peut être inséré dans ledit trocart pour déplacer l'extrémité du fil de façon à s'étendre à l'extérieur de la cavité orale ; un tube de trachéotomie (30) qui est muni d'une extrémité sensiblement conique (40), à l'intérieur de laquelle ladite extrémité dudit fil de traction qui se trouve à l'extérieur de la cavité orale peut être insérée ; **caractérisé par** des moyens d'agrandissement (61) pour empêcher le désengagement du fil de traction (20) depuis ledit tube de trachéotomie (30), ces moyens (61) pouvant être fixés à ladite extrémité dudit fil de traction (20) destinée à être déplacée à l'extérieur de la cavité orale après que ladite extrémité ait été insérée dans ledit tube de trachéotomie (30), grâce à quoi ledit tube de trachéotomie (30) peut être tiré à l'intérieur de la trachée artère, de façon à le faire sortir dudit espace inter-annulaire de l'intérieur vers l'extérieur au moyen d'une traction appliquée audit fil de traction (20) à partir de l'extrémité opposée dudit tube (30).

2. Dispositif selon la revendication 1, **caractérisé en ce qu'**il comprend une canule rigide (1), qui est munie d'un manchon gonflable (2) pour insérer un bronchoscope (6), ladite canule rigide (1) pouvant être configurée de façon à indiquer ledit espace inter-annulaire avec son extrémité libre par transillumination avec le faisceau de lumière projeté par ledit bronchoscope (6).

3. Dispositif selon la revendication 1, **caractérisé en ce que** ledit tube de trachéotomie (30) comprend de plus un élément tubulaire (31) qui comporte un manchon gonflable (32) à une extrémité et qui comporte un élément de pénétration (35) à l'autre extrémité sensiblement conique.

4. Dispositif selon la revendication 3, **caractérisé en ce que** ledit manchon gonflable (32) est relié à un tube de délivrance d'air (32a) qui peut être reçu à l'intérieur dudit élément tubulaire (31) et qui peut être extrait, lorsque l'extrémité de pénétration dudit élément tubulaire (31) quitte la trachée artère, de façon à le relier à un élément de pompage.

5. Dispositif selon la revendication 3, **caractérisé en ce que** ledit élément de pénétration (35) comprend de plus un cône fixe (40) qui est disposé à l'extrémité dudit élément tubulaire (31), un cône souple (41) étant associé sur ledit cône fixe (40), et s'achevant par une pointe de pénétration qui est constituée par une pièce d'insertion en métal.

6. Dispositif selon la revendication 3, **caractérisé en ce que** ledit élément de pénétration (35) est constitué par un cône rigide unique (40) qui est disposé à l'extrémité dudit élément tubulaire (31).

7. Dispositif selon la revendication 3, **caractérisé en ce que** ledit élément de pénétration (35) est constitué par des cônes multiples (55) qui ont des dimensions progressivement de plus en plus grandes et qui sont disposés en séquence par rapport audit fil de traction (20) qui les relie.

8. Dispositif selon la revendication 1, **caractérisé en ce que** lesdits moyens d'agrandissement (61) sont constitués par un premier dispositif de maintien (60) qui est fixé directement à l'extrémité dudit fil de traction et qui peut être inséré dans ledit trocart, un élément en forme de coupelle (61) étant applicable audit premier dispositif de maintien.

9. Dispositif selon la revendication 8, **caractérisé en ce qu'**il comprend de plus une bague élastique (62) qui peut être interposée entre ledit premier dispositif de maintien (60) et ledit élément en forme de coupelle (61).

10. Dispositif selon la revendication 1, **caractérisé en ce que** lesdits moyens d'agrandissement (61) sont constitués en pliant le fil (20) sous la forme d'une lettre U après son insertion à l'intérieur du tube de trachéotomie (30) pour empêcher son extraction.

11. Dispositif selon la revendication 1, **caractérisé en ce que** lesdits moyens d'agrandissement (61) sont constitués par un noeud qui est produit sur ledit fil de traction.

12. Dispositif selon la revendication 1, **caractérisé en ce qu'**il comprend de plus un outil (70) pour tirer ledit fil de traction (20), ledit outil (70) comprenant un corps de base (71) qui est muni d'une plaque (72) qui peut être reposée sur la surface extérieure de la trachée artère, ladite plaque (72) formant un trou de passage (73) pour ledit élément de pénétration (35), une barre de guidage (75) s'étendant à partir de ladite plaque (72) et s'achevant par un élément transversal de butée (76), un élément de coulissement (80) étant disposé de façon à pouvoir coulisser sur ladite barre de guidage (75), et comportant des moyens pour la fixation audit fil de traction (20).

13. Dispositif selon la revendication 12, **caractérisé en ce que** ledit élément de coulissement est de plus muni de creux de saisie (81) pour les doigts de l'opérateur, ledit élément transversal (76) étant approprié pour procurer un appui pour la paume de la main lors de l'application d'une traction audit fil de traction (20).
